# EUROPÄISCHE PATENTSCHRIFT

(11) **EP 3 697 283 B1**
(45) Veröffentlichungstag und Bekanntmachung des Hinweises auf die Patenterteilung: **30.08.2023**
(21) Anmeldenummer: 18796097.6
(22) Anmeldetag: 15.10.2018
(51) Int. Cl.: A61B 1/005

(54) **VERFAHREN ZUR HERSTELLUNG EINES EINFÜHRSCHLAUCHES EINES ENDOSKOPS UND ENDOSKOP MIT EINEM EINFÜHRSCHLAUCH**
METHOD FOR PRODUCING AN ENDOSCOPE INSERTION TUBE AND ENDOSCOPE COMPRISING AN INSERTION TUBE
PROCÉDÉ SERVANT À FABRIQUER UN TUYAU FLEXIBLE D'INSERTION D'UN ENDOSCOPE, ET ENDOSCOPE COMPRENANT UN TUYAU FLEXIBLE D'INSERTION

(30) Priorität: 16.10.2017 DE 102017123975
(43) Veröffentlichungstag der Anmeldung: 26.08.2020
(73) Patentinhaber: Hoya Corporation, Tokyo 160-8347 (JP)
(72) Erfinder: DO, Anh Minh, 86316 Friedberg (DE); VIEBACH, Thomas, 86316 Friedberg (DE)
(74) Vertreter: TBK
(86) Internationale Anmeldenummer: PCT/IB2018/001154
(87) Internationale Veröffentlichungsnummer: WO 2019/077401

(56) Entgegenhaltungen:
- EP-A2- 1 401 526
- EP-B1- 1 401 526
- US-B1- 6 749 560

## Beschreibung

Die vorliegende Erfindung bezieht sich auf ein Verfahren zur Herstellung eines Einführschlauches eines Endoskops und auf ein Endoskop mit einem Einführschlauch.

Ein Endoskop ist ein Gerät, mit dem das Innere von lebenden Organismen, aber auch technischen Hohlräumen untersucht werden kann. Ein wichtiges Teil eines Endoskops ist der flexible Einführschlauch. Die Anforderungen an einen Einführschlauch sind hoch und vielseitig. Einerseits muss er flexibel sein, um in den menschlichen Körper eingeführt werden zu können. Andererseits muss der Einführschlauch bestimmte Steifigkeit besitzen. Bei der Untersuchung muss der Arzt den Einführschlauch anhand des Kontrollkörpers schieben und drehen können. Dabei muss der Einführschlauch so steif sein, dass er nicht geknickt bzw. verdreht wird. Herkömmliche Einführschläuche bedingen daher eine sehr komplexe Aufbauweise und hohe Herstellungskosten, um die genannten Anforderungen zu erfüllen.

Um die Herstellung eines Schlauchelementes für medizinische Zwecke zu vereinfachen und die Herstellkosten zu senken, ist im Stand der Technik die Idee entstanden, ein Schlauchelement für medizinische Zwecke aus einem einzigen harten Rohr herzustellen. Mit einer Laserschneidmaschine werden an dem harten Rohr verschiedene hochpräzise Schnitte erzeugt. Durch die Schnitte wird ein hartes Rohr flexibel, kann aber die Steifigkeit behalten. Die Flexibilität bzw. Steifigkeit des Rohrs kann anhand der Form, der Anordnung und der Größe der Schnitte gesteuert werden.

Die US 6 749 560 B1 offenbart ein Verfahren zur Herstellung eines Einführschlauches eines Endoskops gemäß dem Oberbegriff von Anspruch 1.

Die EP 1 401 526 A1 offenbart eine medizinische Vorrichtung mit einem Körper, in dem eine Vielzahl an Schnitten senkrecht zur Achse vorgesehen sind.

EP 2 777 476 A1 offenbart ein Rohr (30) für ein Endoskop. Das Rohr (30) weist einen Rohrkörper auf, in dem obere Schlitze und untere Schlitze senkrecht zur Rohrachse vorgesehen sind. Die oberen Schlitze und die unteren Schlitze sind zueinander versetzt. Zwischen zwei oberen Schlitzen und zwischen zwei unteren Schlitzen kann ein Wandabschnitt nach innen gebogen werden, um eine Drahtführung zu erzeugen. In einer Variante können zur Erzeugung einer Drahtführung an einer Seite des Rohres separate Schlitze erzeugt werden, die kürzer als die jeweiligen oberen Schlitze und unteren Schlitze sind.

Es ist die Aufgabe der vorliegenden Erfindung, ein Verfahren zur Herstellung eines Einführschlauches eines Endoskops und ein Endoskop mit einem Einführschlauch zu schaffen, die weniger komplex sind und durch die die Kosten noch weiter gesenkt werden können.

In Hinblick auf das Verfahren ist die Aufgabe durch ein Verfahren mit den Merkmalen von Anspruch 1 gelöst. Ein Endoskop mit einem Einführschlauch ist in Anspruch 8 aufgezeigt. Vorteilhafte Weiterbildungen sind Gegenstand der abhängigen Ansprüche.

### Kurzbeschreibung der Zeichnung

Fig. 1 zeigt eine schematische Seitenansicht eines Endoskops.
Fig. 2 zeigt eine ausschnittartige schematische Ansicht eines Einführschlauches.
Fig. 3 zeigt eine ausschnittartige schematische Ansicht eines Teils eines proximalen passiven flexiblen Abschnittes des Einführschlauches,
Fig. 4 zeigt eine ausschnittartige schematische Ansicht eines Übergangsbereiches zwischen dem distalen Abwinkelungsabschnitt und dem proximalen passiven flexiblen Abschnitt des Einführschlauches, wobei ein Führungsfederfixierabschnitt gezeigt ist.
Fig. 5 zeigt eine ausschnittartige perspektivische Ansicht des Führungsfederfixierabschnittes aus Fig. 4 von einer anderen Seite.
Fig. 6 zeigt eine ausschnittartige schematische Ansicht eines Teils des Abwinkelungsabschnittes des Einführschlauches,
Fig. 7 zeigt eine ausschnittartige schematische Ansicht des Teils des Abwinkelungsabschnittes des Einführschlauches, wobei eine Ansicht aus der Richtung eines Pfeiles I aus Fig. 6 gezeigt ist.
Fig. 8 zeigt eine ausschnittartige schematische Ansicht eines Teils des Abwinkelungsabschnittes des Einführschlauches, wobei eine Seilführung gezeigt ist.
Fig. 9 zeigt eine ausschnittartige perspektivische Ansicht der Seilführung aus Fig. 7.
Fig. 10 zeigt eine ausschnittartige schematische Seitenansicht des Abwinkelungsabschnittes des Einführschlauches,
Fig. 11 zeigt eine ausschnittartige schematische Draufsicht des Abwinkelungsabschnittes von Fig. 10.
Die Figuren 12 bis 14 zeigen jeweils eine ausschnittartige perspektivische Ansicht des distalen Endes des Abwinkelungsabschnittes.
Fig. 15 zeigt eine ausschnittartige perspektivische Ansicht der Zugseilverankerung am distalen Ende des Abwinkelungsabschnittes.
Fig. 16 zeigt eine Fig. 15 entsprechende Ansicht von einer anderen Seite.
Fig. 17 zeigt einen Biegeabschnitt eines erfindungsgemässen Ausführungsbeispiels in einer Seitenansicht.
Fig. 18 zeigt eine Draufsicht auf einen distalen Bereich des Biegeabschnittes des erfindungsgemässen Ausführungsbeispiels.
Fig. 19 zeigt eine Querschnittsansicht des Biegeabschnittes des erfindungsgemässen Ausführungsbeispiels.

Nachstehend ist die vorliegende Erfindung detailliert unter Bezugnahme auf die Zeichnungen anhand von Ausführungsbeispielen beschrieben.

### Erstes Ausführungsbeispiel

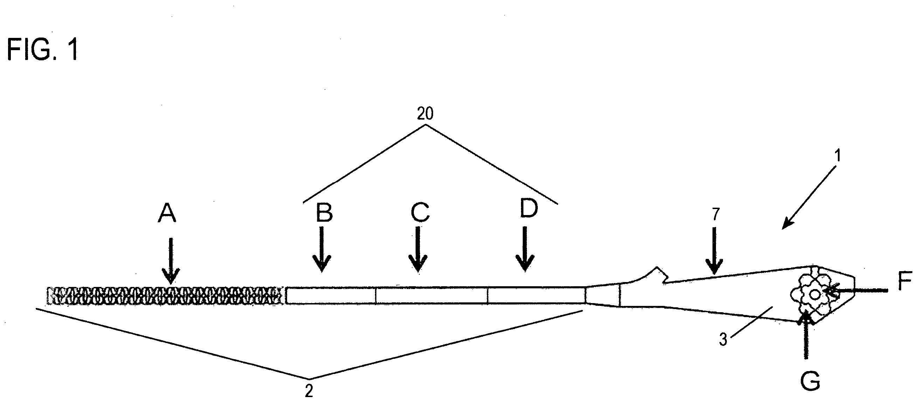

Nachstehend ist unter Bezugnahme auf die Figuren 1 bis 16 ein nicht beanspruchtes erstes Ausführungsbeispiel, das dem Verständnis der vorliegenden Erfindung dient, beschrieben.

Zunächst Fig. 1 zeigt eine schematische Seitenansicht eines Endoskops 1. Wie aus Fig. 1 entnehmbar, hat ein solches Endoskop 1 einen Einführschlauch 2, der an der distalen Seite eines Kontrollkörpers 3 angeordnet ist. Der Kontrollkörper 3 dient als Bedieneinheit des Endoskops 1.

Der Einführschlauch 2 ist ein zylindrisches rohr- oder schlauchartiges Gebilde. Nachstehend ist der Einführschlauch 2 detaillierter in der Richtung, in der er bei einem Patienten eingeschoben wird, beschrieben. Der Einführschlauch 2 wird mit dem distalen Ende voran eingeschoben.

An der distalen Seite besitzt der Einführschlauch 2 einen distalen Abwinkelungsabschnitt A. Der Abwinkelungsabschnitt A kann mittels einem oder mehreren Steuerdrähten (Seilzug oder Seilzüge) relativ zum proximalen Teil des Einführschlauches 2 seitlich abgewinkelt werden. Der Steuerdraht oder Seilzug (nachfolgend nur als Steuerdraht bezeichnet) ist im Inneren des Einführschlauches 2 an einer Innenumfangsfläche des Einführschlauches 2 in Erstreckungsrichtung des Einführschlauches 2 geführt gelagert.

Das distale Ende des Steuerdrahts ist an der distalen Seite des Abwinkelungsabschnittes A verankert. Das proximale Ende des Steuerdrahts ist mit einem im Kontrollkörper 3 angeordneten Steuerelement verbunden. Dieses Steuerelement spannt den Steuerdraht, um eine erwünschte Abwinkelung des Abwinkelungsabschnittes A zu bewerkstelligen.

Proximal vom Abwinkelungsabschnitt A ist der Einführschlauch 2 als ein flexibles Schlauchelement gestaltet, das einen proximalen passiven flexiblen Abschnitt 20 ausbildet. Beim Einschieben des Einführschlauches 2 folgt der flexible Abschnitt 20 dem Abwinkelungsabschnitt A.

In Fig. 1 ist angedeutet, dass der flexible Abschnitt 20 entlang seiner Längsrichtung in Zonen mit unterschiedlicher Flexibilität gestaltet ist. Beispielsweise hat der flexible Abschnitt 20 unter Betrachtung in proximaler Richtung eine erste Zone B, eine zweite Zone C und eine dritte Zone D. Die erste Zone B bildet einen distalen Bereich, die zweite Zone C bildet einen mittleren Bereich und die dritte Zone D bildet einen proximalen Bereich.

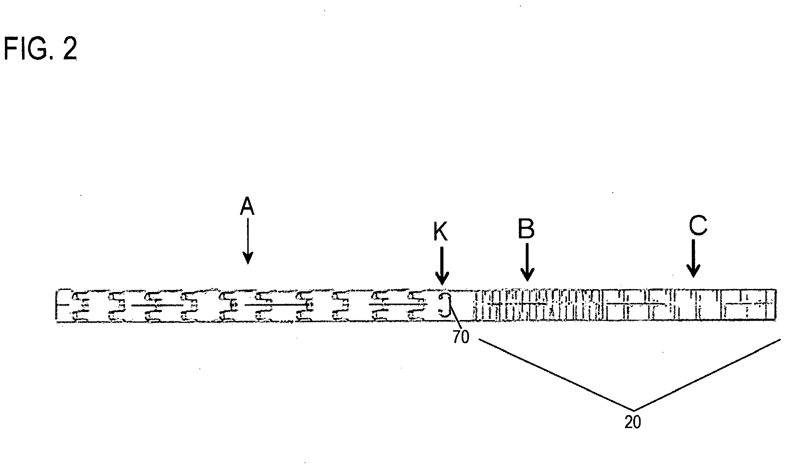

In der ausschnittartigen Darstellung von Fig. 2 ist die dritte Zone D nicht gezeigt.

Zur Vermeidung einer Knickbiegung zwischen dem Abwinkelungsabschnitt A und der ersten Zone B ist die erste Zone B vorzugsweise mit der höchsten Flexibilität unter den Zonen des flexiblen Abschnittes 20 versehen. Da die erste Zone B mit einer sehr hohen Flexibilität ausgestattet ist, ergibt sich kein abrupter Übergang der Flexibilität zwischen dem Abwinkelungsabschnitt A und der ersten Zone B.

Die zweite Zone C hat eine geringere Flexibilität als die erste Zone B. Die dritte Zone D hat eine wiederum geringere Flexibilität als die zweite Zone C.

Der erfindungsgemäße Einführschlauch 2 ist aus einem Stück gebildet. Das heißt, am Übergang vom Abwinkelungsabschnitt A zum flexiblen Abschnitt 20 sind nicht zwei Elemente zusammengefügt. Somit sind der distale Abwinkelungsabschnitt A und der proximale passive flexiblen Abschnitt 20 mit den drei Zonen A, B und C aus einem einzigen Rohr oder Schlauch gebildet.

An der proximalen Seite ist der Einführschlauch 2 am distalen Ende des Kontrollkörpers 3 fixiert. Der Einführschlauch 2 kann am Kontrollkörper 3 z.B. durch einen Feststellring, einen Dichtungsring oder direkt fixiert sein. Der Einführschlauch 2 kann am Kontrollkörper 3 z.B. angeklebt oder angeschraubt sein. Der Kontrollkörper 3 hat ein erstes Steuerrad F als ein erstes Steuerelement zum Steuern eines Steuerdrahtes oder Seilzugs und ein zweites Steuerrad G als ein zweites Steuerelement zum Steuern eines Steuerdrahtes oder Seilzugs. Das erste Steuerrad F kann durch Ziehen eines Steuerdrahtes oder Seilzugs den Abwinkelungsabschnitt A in einer ersten Ebene abwinkeln (z.B. zum Betrachter hin und vom Betrachter weg in Fig. 1). Das zweite Steuerrad G kann durch Ziehen eines Steuerdrahtes oder Seilzugs den Abwinkelungsabschnitt A in einer zweiten Ebene, die senkrecht zur ersten Ebene ist, abwinkeln (z.B. in Fig. 1 nach oben und unten).

Der Abwinkelungsabschnitt A kann z.B. um 200 - 270 Grad abgewinkelt werden. Dies ist für die meisten Anwendungen ausreichend. In einer Spezialform kann der Abwinkelungsabschnitt A sogar um 300 Grad abgewinkelt werden.

Nachstehend ist der erfindungsgemäße Einführschlauch 2 und seine Herstellung detaillierter beschrieben.

Der gesamte Einführschlauch 2 ist aus einem einzigen Rohrelement oder Schlauchelement (nachstehend ist dieses einfach als Rohrelement bezeichnet) gebildet. Das Rohrelement ist ein Rohr aus vorzugsweise relativ hartem Material. Besonders bevorzugt ist ein Rohr aus Edelstahl. Es kann aber auch ein Rohr aus hartem Kunststoff angewendet werden. Im Prinzip kann aber jedes für medizinische Zwecke anwendbare Material genutzt werden.

Durch eine Laserschneidmaschine werden im Rohrelement Schnitte vorgesehen, wie dies nachstehend detaillierter erläutert ist. Nach dem Vorsehen der Schnitte werden bestimmte Teilabschnitte des Rohrelementes gebogen, wie dies nachstehend detaillierter erläutert ist. Die Herstellung des Grundkörpers des gesamten Einführschlauches 2 macht keine weiteren Verfahrensschritte außer dem Vorsehen von Schnitten und dem Biegen erforderlich. Danach kann der Grundkörper des Einführschlauches 2 mit einem Steuerdraht versehen werden und einem Mantelelement ummantelt werden.

Nachstehend sind die einzelnen Abschnitte des Einführschlauches 2 genauer beschrieben.

### Flexibler Abschnitt 20

Der flexible Abschnitt 20 bildet den proximalen Teil des erfindungsgemäßen Einführschlauches 2. Der flexible Abschnitt 20 hat die drei Zonen B, C und D mit jeweils unterschiedlicher Flexibilität.

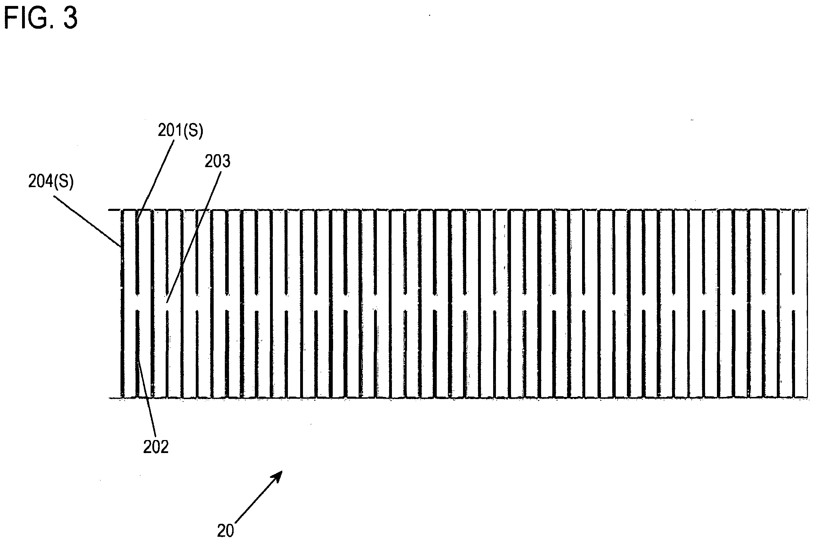

Fig. 3 zeigt eine Möglichkeit zur Ausbildung einer der drei Zonen B, C und D des flexiblen Abschnittes 20 in einer Seitenansicht.

Der flexible Abschnitt 20 ist mit einer Vielzahl an Schnitten S versehen, die senkrecht zur Achse des flexiblen Abschnittes 20 ausgeführt sind. Genauer gesagt sind die Schnitte S so ausgeführt, dass ein Schnitt 201 von oben durch das Rohrelement senkrecht zur Achse des Rohrelements bis zu einer Tiefe erfolgt, die vor dem Mittelachsenbereich endet. Ferner erfolgt ein Schnitt 202 von unten durch das Rohrelement senkrecht zur Achse des Rohrelements bis zu einer Tiefe, die ebenfalls vor dem Mittelachsenbereich endet. Die Schnitte 201 und 202 liegen auf einer Ebene und ihre Enden stehen einander über einen belassenen Zwischenraum 203 gegenüber. Der Zwischenraum 203 ist ein nicht geschnittener Zwischenraum im Mittelachsenbereich des Rohrelements.

Darüber hinaus erfolgt ähnlich wie bei den Schnitten 201 und 202 ein Schnitt 204 von einer (z.B. linken) Seite (der Schnitt 204 zeigt einen Schnitt von der Seite des Betrachters) durch das Rohrelement senkrecht zur Achse des Rohrelements bis zu einer Tiefe, die vor dem Mittelachsenbereich endet. Ferner erfolgt ein Schnitt von der entgegengesetzten (z.B. rechten) Seite (dieser Schnitt ist in Fig. 3 nicht gezeigt, da er jenseits der Bildebene liegt) durch das Rohrelement senkrecht zur Achse des Rohrelements bis zu einer Tiefe, die ebenfalls vor dem Mittelachsenbereich endet. Diese Schnitte liegen auch auf einer Ebene und ihre Enden stehen einander ebenfalls über einen belassenen Zwischenraum gegenüber. Dieser Zwischenraum ist ebenfalls ein nicht geschnittener Zwischenraum im Mittelachsenbereich des Rohrelements.

Der Zwischenraum 203 zwischen den Schnitten 201 und 202 und der Zwischenraum zwischen Schnitt 204 und seinem zugehörigen Schnitt der entgegengesetzten Seite sind um 90 Grad entlang der Umfangsrichtung des Rohrelements versetzt.

Die Schnitte 201 und 202 und die Schnitte 204 und sein zugehöriger Schnitt der entgegengesetzten Seite sind benachbart zueinander und wechseln einander über die Länge der jeweiligen Zone im flexiblen Abschnitt 20 ab, siehe Fig. 3. D

Somit ist der flexiblen Abschnitt 20 lateral zu seiner Längsachse um die Zwischenräume biegbar.

Die einzelnen Zonen B, C und D unterscheiden sich dadurch, dass die Abstände der Schnitte S in Längsrichtung und somit die Dichte der Schnitte S unterschiedlich gestaltet sind.

In der Zone B ist der Abstand der Schnitte S am geringsten. Somit ist in der Zone B die Dichte der Schnitte S am höchsten.

In der Zone C ist der Abstand der Schnitte S größer als in der Zone B. In der Zone D ist der Abstand der Schnitte S größer als in der Zone C.

Somit ist die Flexibilität und die Biegbarkeit in der Zone B höher als in der Zone C. Ferner ist die Flexibilität und die Biegbarkeit in der Zone C höher als in der Zone D. Anders ausgedrückt nehmen die Flexibilität und die Biegbarkeit der jeweiligen Zonen am flexiblen Abschnitt 20 in proximaler Richtung ab.

Die Zone D ist an der proximalen Seite mit einem Bereich versehen, der nicht mit Schnitten versehen ist. Dieser Bereich bildet einen Übergang zum Kontrollkörper J.

### Übergang vom Abwinkelungsabschnitt A zum flexiblen Abschnitt 20

Der Übergangsbereich vom Abwinkelungsabschnitt A zum flexiblen Abschnitt 20 ist in Fig. 2 als Bereich K angedeutet. In diesem Bereich K endet der Abwinkelungsabschnitt A. Anders ausgedrückt befindet sich distal vom Bereich K das erste d.h. am weitesten proximal befindliche Glied des Abwinkelungsabschnittes A.

Wie in Fig. 2 gezeigt, ist in diesem Bereich K die Wandfläche des Rohrelements durch einen Schnitt 70 in der Form eines umgekehrten Buchstaben C geschnitten. Anders ausgedrückt, ist der Schnitt 70 im Rohrelement in der Form eines unvollständigen Kreises geschnitten. Der Kreis des Schnittes 70 ist an der distalen Seite nicht durchgeschnitten. Die nicht durchgeschnittene distale Seite des Schnittes 70 bildet ein Scharnier 71 für eine Lasche 72. Die Lasche 72 hat ein unteres Ohr 73, ein oberes Ohr 74 und ein Laschenmittelstück 75. An einer oberen Seite des Laschenmittelstücks 75 grenzt das untere Ohr 73 an. An einer unteren Seite des Laschenmittelstücks 75 grenzt das obere Ohr 74 an.

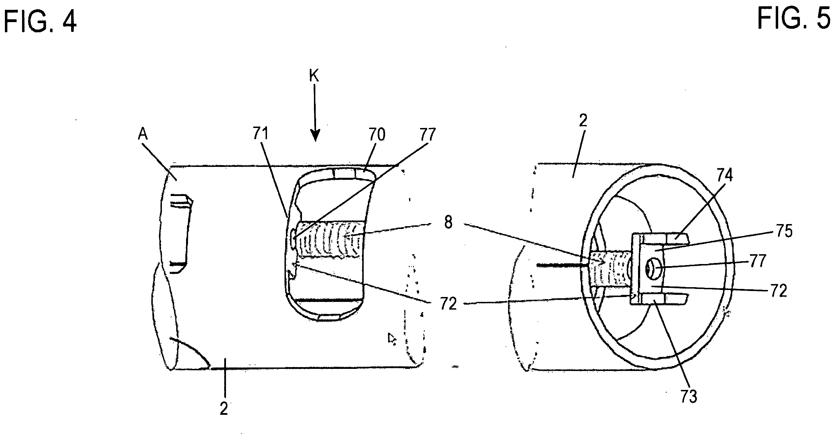

Die Lasche 72 wird wie folgt hergestellt. Der Ort des Schnittes 70 wird festgelegt. In der Mitte des Schnittes 70 wird ein Loch 77 geschnitten. Der Schnitt 70 wird per Laser wie in Fig. 2 gezeigt gebildet. Das Laschenmittelstück 75 wird von der Rückseite, d.h. von der Innenseite des Rohrelements durch einen Stempel abgestützt. Das untere Ohr 73 wird relativ zum Laschenmittelstück 75 um 90 Grad nach innen gebogen. Die Biegelinie des Ohres 73 relativ zum Laschenmittelstück 75 verläuft dabei parallel zur Achse des Rohrelements (in Figuren 2 und 4 in der nach links und nach rechts weisenden Richtung). Das obere Ohr 74 wird ebenfalls relativ zum Laschenmittelstück 75 um 90 Grad nach innen gebogen. Die Biegelinie des Ohres 74 relativ zum Laschenmittelstück 75 verläuft ebenfalls parallel zur Achse des Rohrelements. Danach wird das Laschenmittelstück 75 um 90 Grad nach innen gebogen. Die Biegelinie des Laschenmittelstücks 75 relativ zum Rohrelement verläuft in der senkrechten Schnittebene zur Achse des Rohrelements (in Figuren 2 und 4 in der nach oben und nach unten weisenden Richtung). Anders ausgedrückt wird das Laschenmittelstück 75 am Scharnier 71 um 90 Grad nach innen gebogen. Das Laschenmittelstück 75 wird insbesondere so weit nach innen gebogen, bis eine distale Seitenkante des unteren Ohres 73 und eine distale Seitenkante des oberen Ohres 74 am Innenumfang des Rohrelements anliegen, siehe Fig. 5).

Die Lasche 72 dient als Abstützung einer Führungsfeder 8. Insbesondere bildet die proximale Fläche des Laschenmittelstücks 75 eine Anschlagfläche für das distale Ende der Führungsfeder 8. Die beiden Ohren 73, 74 stützen das Laschenmittelstück 75 und nehmen von der Führungsfeder 8 wirkende Drückkräfte auf und leiten diese an die Innenumfangsfläche des Rohrelements weiter.

Das Laschenmittelstück 75 besitzt das zentrische Loch 77. Das Loch 77 hat einen größeren Durchmesser als ein Steuerdraht und einen kleineren Durchmesser als die Führungsfeder 8. Der Steuerdraht wird im flexiblen Abschnitt 20 in der Führungsfeder 8 geführt und durchläuft das Loch 70 und erstreckt sich weiter in den Abwinkelungsabschnitt A hinein.

Im Bereich K sind Laschen 72 in der Anzahl der verwendeten Steuerdrähte (im vorliegenden Ausführungsbeispiel: vier) vorgesehen. Die Laschen 72 sind in Umfangsrichtung des Rohrelements gleichmäßig verteilt.

### Abwinkelungsabschnitt A

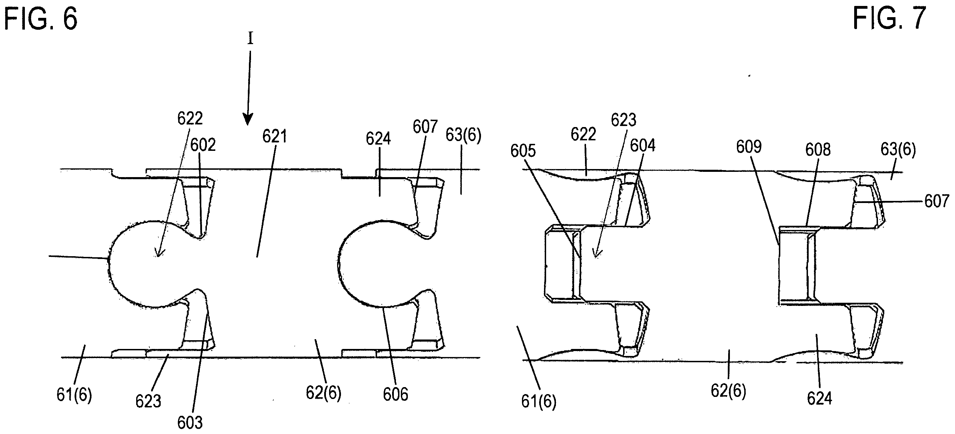

Der genauere Aufbau des Abwinkelungsabschnittes A ist in den Figuren 6 bis 11 gezeigt.

Der Abwinkelungsabschnitt A hat einzelne Gelenkglieder 6, die in Längsrichtung des Abwinkelungsabschnittes A angeordnet sind. Die einzelnen Gelenkglieder 6 sind relativ zueinander schwenkbar. In den Figuren 6 und 7 sind drei hintereinander angeordnete Gelenkglieder 6 gezeigt: ein Gelenk 61, proximal vom Gelenk 61 ein Gelenk 62 und proximal vom Gelenk 62 ein Gelenk 63.

Die Gelenkglieder 6 sind zueinander gleich gestaltet mit Ausnahme des am weitesten distal befindlichen Gelenkglieds 6 und des am weitesten proximal befindlichen Gelenkglieds 6.

Der Aufbau des jeweiligen Gelenkglieds 6 ist nachstehend anhand Gelenkglied 62 erörtert.

Das Gelenkglied 62 ist als ein Rohrabschnitt des genannten Rohrelements durch Laserschneiden ausgebildet. Das Gelenkglied 62 besitzt am Umfang des Rohrelements distale Begrenzungslinien 601, 602, 603, 604 und 605 und proximale Begrenzungslinien 606, 607, 608 und 609.

Die einzelnen distalen Begrenzungslinien setzen sich zusammen aus einer kreisartig geformten Kopflinie 601, zwei Halslinien 602, zwei Schulterlinien 603, zwei Armlinien 604 und einer Armendlinie 605. Genauer gesagt ist die distale Seite des Gelenkglieds 62 folgendermaßen gebildet. Die kreisartig geformte Kopflinie 601 bildet einen unvollständigen Kreis, der an der proximalen Seite an jeder Seite in eine Halslinie 602 übergeht. An jeder der beiden Halslinien 602 schließt sich eine Schulterlinie 603 an, die annähernd senkrecht zur Achse des Rohrelements verläuft. An jeder der beiden Schulterlinien 603 schließt sich eine Armlinie 604 an, die annähernd parallel zur Achse des Rohrelements in die distale Richtung verläuft. Die beiden distalen Enden der Armlinien 604 sind durch eine Armendlinie 605 verbunden, die wieder senkrecht zur Achse des Rohrelements verläuft.

Dadurch hat das Gelenkglied 62 einen Hauptkörper 621, von dem zur distalen Seite hin ein erster Kopf 622, ein erster Arm 623, ein zweiter Kopf 622 und ein zweiter Arm 623 jeweils um 90 Grad entlang einer gedachten Umfangslinie, die senkrecht zur Achse des Gelenkglieds 62 verläuft, vorragen. Somit erstrecken sich die Köpfe 622, 622 in einer ersten gedachten Ebene. Die Arme 623, 623 erstrecken sich in einer zweiten gedachten Ebene, die um 90 Grad versetzt zur ersten gedachten Ebene ist. Die beiden Köpfe 622, 622 des Gelenkgliedes 62 bilden eine Schwenkachse für das distal von ihnen befindliche Gelenkglied 61.

Jeder Kopf 622 ist an der distalen Seite durch eine Kopflinie 601 gebildet. Zwischen dem Kopf 622 und dem Hauptkörper 621 ist eine Verengung durch die Halslinien 602 gebildet. Der jeweilige Kopf 622 ragt weiter in der distalen Richtung vor als der jeweilige Arm 623.

Die einzelnen proximalen Begrenzungslinien setzen sich zusammen aus einer gebogenen Fußlinie 606, zwei Bodenlinien 607, zwei geraden Fußlinien 608 und einer Bauchlinie 609. Genauer gesagt ist die proximale Seite des Gelenkglieds 62 folgendermaßen gebildet. Die gebogene Fußlinie 606 bildet einen unvollständigen Kreis, der an der proximalen Seite offen ist. An den offenen Enden des unvollständigen Kreises geht die gebogene Fußlinie 606 jeweils in die Bodenlinie 607 über, die jeweils annähernd senkrecht zur Achse des Rohrelements verläuft.

An jeder der beiden Bodenlinien 607 schließt sich eine gerade Fußlinie 608 an, die annähernd parallel zur Achse des Rohrelements in die distale Richtung verläuft. Die beiden distalen Enden der geraden Fußlinien 608 sind durch eine Bauchlinie 609 verbunden, die wieder senkrecht zur Achse des Rohrelements verläuft.

Dadurch hat das Gelenkglied 62 an der proximalen Seite des Hauptkörpers 621 zwei Füsse 624, die sich in proximaler Richtung erstrecken. Jeder Fuß 624 hat in Erstreckungsrichtung eine gerade Seite an der geraden Fußlinie 608 und eine gekrümmte Seite an der gebogenen Fußlinie 606.

In dem Bereich zwischen den beiden geraden Fußlinien 608 ist ein Arm des proximal befindlichen Gelenkgliedes 63 in Längsrichtung verschiebbar angeordnet. In dem Bereich zwischen den beiden gebogenen Fußlinien 606 wird ein Kopf des proximal befindlichen Gelenkgliedes 63 in Längsrichtung unbeweglich gehalten. Allelfalls eine geringfügige Bewegung aufgrund eines Spieles zwischen dem Innenumfang der gebogenen Fußlinie und dem Außenumfang der kreisartig geformten Kopflinie ist möglich.

Im nicht gebogenen Zustand des Abwinkelungsabschnittes A ist die Bauchlinie 609 von der Armendlinie 605 des proximal befindlichen Gelenkgliedes 63 beabstandet, wie dies in Fig. 7 gezeigt ist. Die Armendlinie 605 und die Bauchlinie 609 des proximal befindlichen Gelenkgliedes 63 sind parallel zueinander.

Im nicht gebogenen Zustand des Abwinkelungsabschnittes A ist die Bodenlinie 607 von der Schulterlinie 603 des proximal befindlichen Gelenkgliedes 63 beabstandet, wie dies in Fig. 7 gezeigt ist. Die Bodenlinie 607 und die Schulterlinie 603 des proximal befindlichen Gelenkgliedes 63 können parallel zueinander oder annähernd parallel zueinander sein oder leicht winklig zueinander sein, wie dies in Fig. 7 gezeigt ist. Zwischen der Bodenlinie 607 und der Schulterlinie 603 des proximal befindlichen Gelenkgliedes 63 ist nicht nur eine einfache Schnittlinie erzeugt worden, sondern das Material des Rohrelementes ist als ein viereckiges Stück herausgeschnitten worden.

Ein jeweiliger Kopf 622 bildet einen Kupplungsabschnitt, der mit einem benachbarten Gelenkglied 6 gekuppelt ist. Die Füße 624 bilden einen Führungsabschnitt, der mit einem benachbarten Gelenkglied 6so in Eingriff steht, dass eine axiale Bewegung der Gelenkglieder 6 zueinander ermöglicht ist.

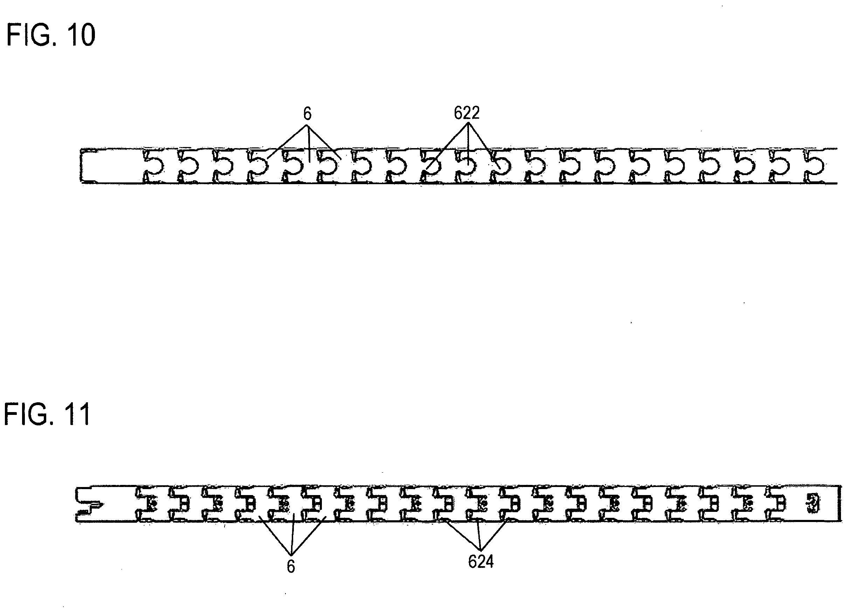

Fig. 10 zeigt eine Draufsicht auf den Abwinkelungsabschnitt A mit den jeweiligen Gelenkgliedern 6. In der Draufsicht sind die Köpfe 622 der Gelenkglieder 6 zu sehen.

Fig. 11 zeigt eine Seitenansicht des Abwinkelungsabschnittes A mit den jeweiligen Gelenkgliedern 6. In der Seitenansicht sind die Füße 624 der Gelenkglieder 6 zu sehen.

Das am weitesten distal befindliche Gelenkglied 6 hat keinen Kopf und ist in den Figuren 2 und 10 bis 14 gezeigt.

Das am weitesten proximal befindliche Gelenkglied 6 hat keinen Fuß und ist in den Figuren 2, 4 und 11 gezeigt.

Im Ausführungsbeispiel kann der Abwinkelungsabschnitt A in zwei Abwinkelungsrichtungen abgewinkelt werden, nämlich in den Figuren 6 und 7 (und Fig. 10) nach oben und unten, wobei die jeweiligen Köpfe 622 der Gelenkglieder 6 Biegeachsen der Gelenkglieder 6 bilden. Anders ausgedrückt, ist der Abwinkelungsabschnitt A in Fig. 10 nach oben und unten schwenkbar. In der Darstellung von Fig. 11 ist der Abwinkelungsabschnitt A zum Betrachter hin und vom Betrachter schwenkbar.

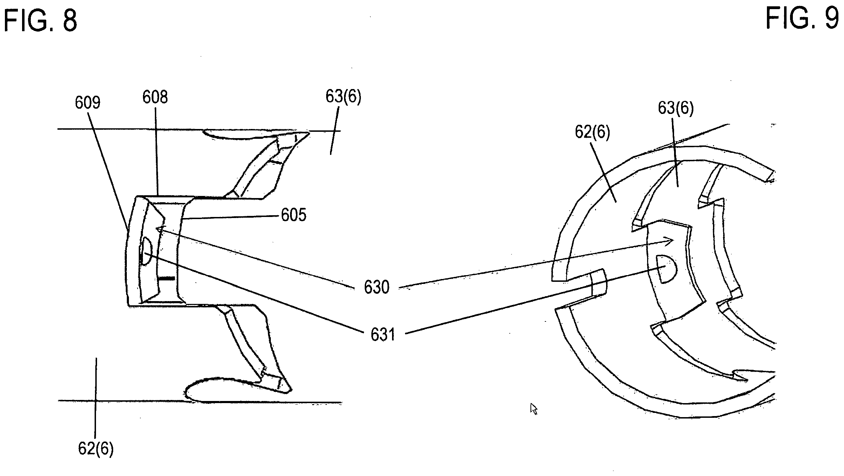

Wie dies in den Figuren 8 und 9 gezeigt ist, bildet die Bauchlinie 609 einen Scharnierabschnitt für eine Seilführungslasche 630. Die Seilführungslasche 630 erstreckt sich von der Bauchlinie 609. Für die Seilführungslasche 630 wird ein Materialabschnitt genommen, der sich entlang der geraden Fußlinien 608 bis zur Armendlinie 605 des proximal befindlichen Gelenkgliedes 63 erstreckt. Die Seilführungslasche 630 ist an der Bauchlinie 609 angelenkt und um 90 Grad nach innen gebogen. Die Seilführungslasche 630 besitzt ein zentrisches Loch 631. Das Loch 631 hat einen größeren Durchmesser als der Steuerdraht.

Jedes der Gelenkglieder 6 besitzt die Seilführungslaschen 630 mit dem Loch 631 so, dass die Seilführungslaschen 630 für einen spezifischen Steuerdraht in Längsrichtung des Abwinkelungsabschnittes A hintereinander angeordnet sind. Die Seilführungslaschen 630 dienen als Führungsvorsprünge, an denen ein Steuerdraht abgestützt ist Somit führen die Seilführungslaschen 630 den ihnen zugewiesenen Steuerdraht durch den Abwinkelungsabschnitt A.

Die Gelenkglieder 6 können am Abwinkelungsabschnitt A so angeordnet sein, dass ihre Köpfe in die proximale Richtung weisen, wie dies in Fig. 10 gezeigt ist. Alternativ können die Gelenkglieder 6 am Abwinkelungsabschnitt A so angeordnet sein, dass ihre Köpfe in die distale Richtung weisen, wie dies in Fig. 6 angedeutet ist.

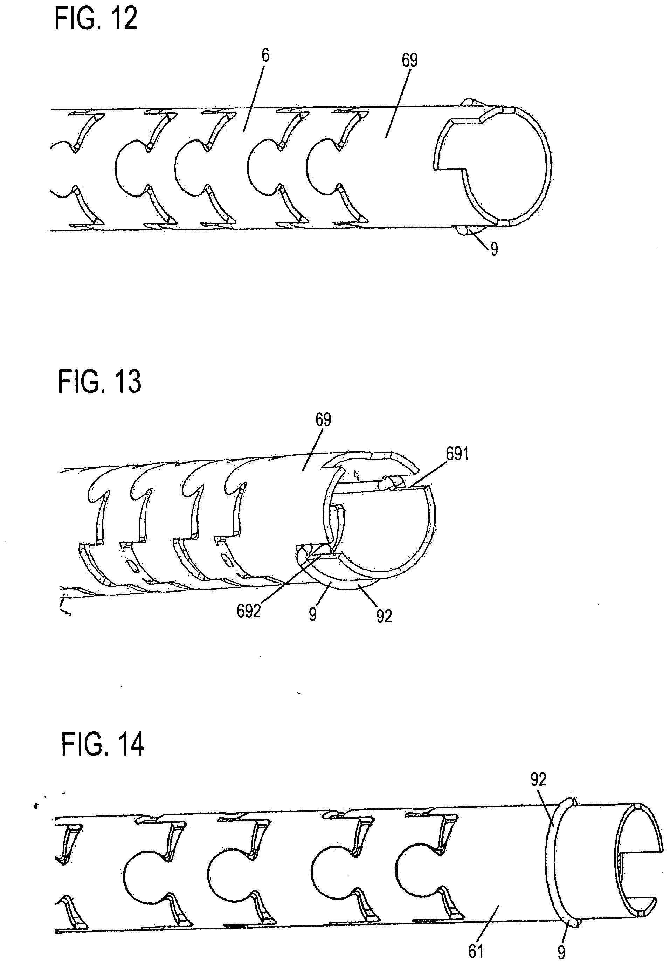

Das distale Ende des Abwinkelungsabschnittes A ist in den Fig. 12 bis 14 gezeigt. In den in den Fig. 12 bis 14 ist das am weitesten an der distalen Seite befindliche Gelenkglied 69 des Abwinkelungsabschnittes A erkennbar. In diesem am weitesten an der distalen Seite befindlichen Gelenkglied 69 ist die distale Seite des Steuerdrahtes 9 verankert. Der Steuerdraht 9 erstreckt sich vom Kontrollkörper 3 bis zum am weitesten an der distalen Seite befindlichen Gelenkglied 69 des Abwinkelungsabschnittes A.

### Befestigung des Steuerdrahtes

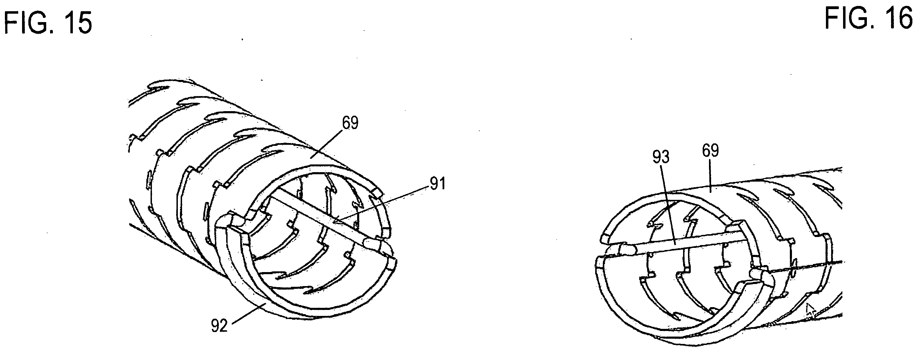

Die genaue Befestigung des Steuerdrahtes 9 ist in den Figuren 15 und 16 gezeigt.

Der Steuerdraht 9 ist im Kontrollkörper 3 am Steuerrad G befestigt. Wenn das Steuerrad G in eine Spannrichtung gedreht wird, wird der Steuerdraht 9 gespannt. Wenn das Steuerrad G in die zur Spannrichtung entgegengesetzte Entlasungsrichtung gedreht wird, wird der Steuerdraht 9 entlastet.

Der Steuerdraht 9 erstreckt sich vom Kontrollkörper 3 kommend im Einführschlauch 2 verlaufend bis zum Gelenkglied 69 und bildet einen ersten Abschnitt 91. Dieser erste Abschnitt 91 des Steuerdrahtes 9 läuft am Innenumfang des Einführschlauches 2 entlang. Dieser erste Abschnitt 91 des Steuerdrahtes 9 ist anhand Bezugszeichen 91 in Fig. 15 gezeigt. An der distalen Seite des Gelenkgliedes 69 ist ein die Umfangswand des Gelenkgliedes 69 durchdringender Schlitz 691 ausgebildet (siehe Fig. 13), der sich in der Längsrichtung des Gelenkgliedes 69 erstreckt. Ein weiterer ähnlicher Schlitz 692 ist an der distalen Seite des Gelenkgliedes 69 dem Schlitz 691 diametral gegenüberliegend vorgesehen.

Der Steuerdraht 9 erstreckt sich am Innenumfang des Gelenkgliedes 69 in die distale Richtung und durchdringt den Schlitz 691 nach außen, ist am Außenumfang des Gelenkgliedes 69 in Umfangsrichtung des Gelenkgliedes 69 bis zum Schlitz 692 gewunden, durchdringt den Schlitz 692 nach innen, und erstreckt sich am Innenumfang des Gelenkgliedes 69 in die proximale Richtung bis hin zum Steuerrad G im Kontrollkörper 3.

Der Steuerdraht 9 ist somit in einen ersten Abschnitt 91, der sich vom Steuerrad G im Kontrollkörper 3 bis zum Schlitz 691 erstreckt, einen zweiten Abschnitt 92, der sich vom Schlitz 691 am Außenumfang des Gelenkgliedes 69 in Umfangsrichtung des Gelenkgliedes 69 bis zum Schlitz 692 erstreckt, und einen dritten Abschnitt 93 geteilt, der sich vom Schlitz 692 bis zum Steuerrad G im Kontrollkörper 3 erstreckt.

Durch Drehen des Steuerrades G in die Spannrichtung wird der Steuerdraht 9 gespannt und somit der Abwinkelungsabschnitt A abgewinkelt, da der am Gelenkglied 69 verankerte dritte Abschnitt 93 in die proximale Richtung gedrängt wird. Der dritte Abschnitt 93 des Steuerdrahtes 9 bildet somit einen distalen Verankerungsabschnitt des Steuerdrahtes 9.

### Herstellverfahren

Der erfindungsgemäße Einführschlauch 2 wird durch ein einziges Rohrelement hergestellt, das per Laser geschnitten wird. Das Rohrelement ist aus einem relativ harten Material, wie z.B. Edelstahl oder auch geeigneter harter Kunststoff hergestellt. Durch die Schnitte wird das zunächst harte Rohrelement flexibel, behält aber seine Steifigkeit.

Die Schnitte erzeugen die jeweiligen seitlichen Einschnitte S im proximalen passiven flexiblen Abschnitt 20, das Loch 77, den Schnitt 70 im Übergangsbereich K, das Loch 631, die jeweiligen Gelenkglieder 6 im distalen Abwinkelungsabschnitt A und die Schlitze 691, 692. Diese Reihenfolge ist nicht als Einschränkung aufzufassen. Z.B. können die Schlitze 691, 692 vor den Gelenkgliedern 6 geschnitten werden. Außerdem kann die Reihenfolge der Schnitte auch umgekehrt werden.

Die Flexibilität und auch die Steifigkeit des Rohrelementes können anhand der Form, der Anordnung und der Größe der Schnitte gesteuert werden.

Der Ort der jeweiligen Schnitte kann zuvor berechnet und vorbestimmt werden. In einer programmierbaren Laserschneidmaschine können die vorgegebenen Daten für die jeweiligen Schnitte eingegeben werden, um den Einführschlauch 2 automatisch zu erzeugen.

Die einzelnen Gelenkglieder 6 werden vollständig ausgeschnitten und bilden voneinander körperlich getrennte Körper, die lediglich formschlüssig verbunden sind.

Nach dem Laserschneiden des Rohrelementes werden die Laschen 72 und die Seilführungslaschen 630 nach innen gebogen. Somit ist der Rohkörper für den Einführschlauch 2 fertiggestellt.

In diesem Rohkörper für den Einführschlauch 2 kann nun der Steuerdraht 9 eingeführt und befestigt werden. Der Rohkörper für den Einführschlauch 2 kann am Kontrollkörper 3 befestigt werden. Ferner kann auf den Rohkörper für den Einführschlauch 2 ein den Rohkörper für den Einführschlauch 2 umgebender Überzug aus vorzugsweise Metall zur Abschirmung der elektrischen Steuerung und auf diesen ein elastischer Mantel aus Kunststoff oder Gummi aufgezogen werden. Der elastische Mantel aus Kunststoff oder Gummi kann einem thermischen Schrumpfen ausgesetzt werden.

### Zweites Ausführungsbeispiel

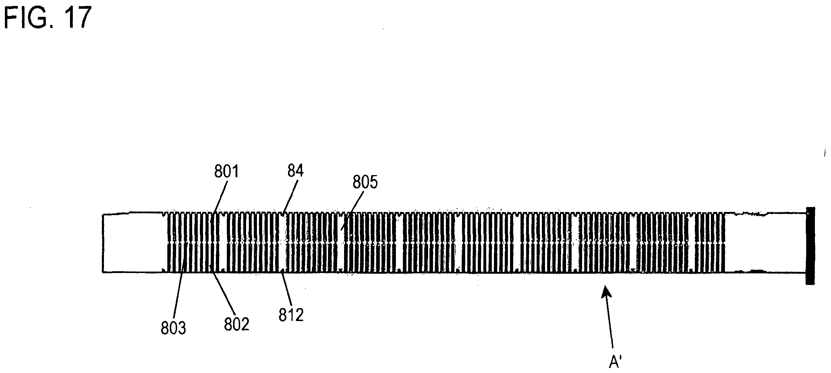

Nachstehend ist unter Bezugnahme auf die Figuren 17 - 19 ein zweites Ausführungsbeispiel der vorliegenden Erfindung beschrieben.

Im ersten Ausführungsbeispiel sind im Biegeabschnitt die einzelnen Gelenke anhand von Schnitten so gebildet, dass sie in Erstreckungsrichtung des Endoskops Vorsprünge und Vertiefungen haben. Die Vorsprünge sitzen in den Vertiefungen des benachbarten Gelenks, um eine Schwenkbewegung des Gelenks zu gestatten. Anders ausgedrückt, sind im ersten Ausführungsbeispiel die einzelnen Gelenke formschlüssig verbunden.

Die Figuren 17 - 19 den Abwinkelungsabschnitt A' als Biegeabschnitt des zweiten Ausführungsbeispiels. Im zweiten Ausführungsbeispiel sind lediglich gerade Schnitte 801, 802, 811, 812 im Biegeabschnitt A' vorgesehen.

Der Biegeabschnitt A' ist mit einer Vielzahl an Schnitten 801, 802 versehen, die senkrecht zur Achse des Biegeabschnittes A' ausgeführt sind. Genauer gesagt sind die Schnitte 801, 802 so ausgeführt, dass ein Schnitt 801 von oben durch das Rohrelement senkrecht zur Achse des Rohrelements bis zu einer Tiefe erfolgt, die vor dem Mittelachsenbereich endet. Ferner erfolgt ein Schnitt 802 von unten durch das Rohrelement senkrecht zur Achse des Rohrelements bis zu einer Tiefe, die ebenfalls vor dem Mittelachsenbereich endet. Die Schnitte 801 und 802 liegen auf einer Ebene und ihre Enden stehen einander über einen belassenen Zwischenraum 803 gegenüber. Der Zwischenraum 803 ist ein nicht geschnittener Zwischenraum im Mittelachsenbereich des Rohrelements. Die Schnitte 801 sind parallel zueinander. Die Schnitte 802 sind in analoger Weise ebenfalls parallel zueinander.

Die geraden Schnitte 801, 802 fungieren als Gelenk und ermöglichen die Biegebewegung des Biegeabschnittes A'.

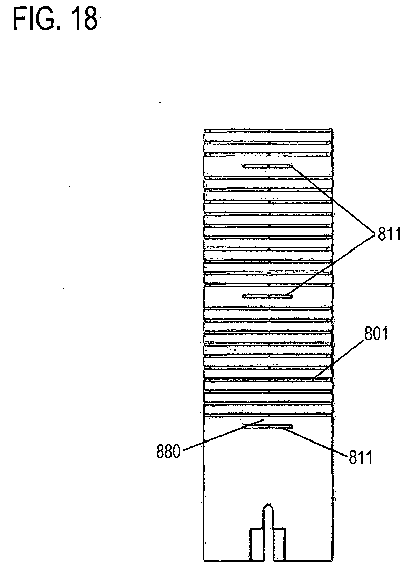

Eine vordefinierte Anzahl an in Längsrichtung des Biegeabschnittes A' hintereinander liegender Schnitte 801 (und natürlich analog 802) sind zu einer Gruppe zusammengefasst. In den Fig. 18 gehören jeweils 10 Schnitte 801 zu einer Gruppe, wobei die Anzahl an Schnitten 801, 802 je Gruppe beliebig geeignet gewählt werden kann. Je mehr Schnitten 801, 802 eine Gruppe hat, desto größer ist der Biegewinkel in dem Bereich dieser Gruppe.

Die jeweilige Gruppe an Schnitten 801, 802 ist in Längsrichtung des Biegeabschnittes A' von einem Ringabschnitt 805 mit kurzen Schnitten 811, 812 begrenzt.

Genauer gesagt sind die kurzen Schnitte 811, 812 so ausgeführt, dass ein kurzer Schnitt 811 von oben durch das Rohrelement senkrecht in Richtung zur Achse des Rohrelements bis zu einer sehr kurzen Tiefe erfolgt, wie dies in Fig. 17 gezeigt ist. Diese kurze Tiefe kann z.B. ein Zehntel bis ein Zwanzigstel des Durchmessers des Rohrelementes betragen. Somit ergibt sich eine kurze Länge des jeweiligen Schnittes 811, 812, wie dies in Fig. 18 gezeigt ist, in der kurze Schnitte 811 von oben gezeigt sind. Die Länge der kurzen Schnitte 811, 812 kann beliebig geeignet gewählt werden.

Ferner erfolgt ein jeweiliger kurzer Schnitt 812 von unten in ähnlicher Weise wie beim den kurzen Schnitten 811 von oben. Die Schnitte 811 sind parallel zueinander. Die Schnitte 812 sind in analoger Weise ebenfalls parallel zueinander.

Die kurzen Schnitte 811 und 812 bilden jeweils ein Paar und liegen jeweils auf einer Ebene und ihre Enden stehen einander über einen belassenen Zwischenraum gegenüber, der den Ringabschnitt 805 ausbildet. Der Ringabschnitt 805 ist ein Abschnitt des Rohrelements, der lediglich ein Paar an kurzen Schnitte 811, 812 aufweist.

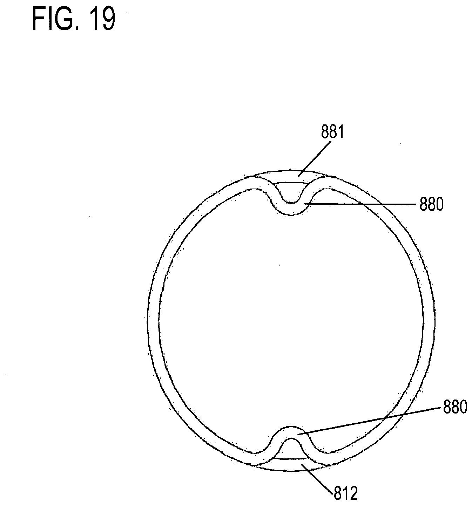

Das Material des Rohrelements, das sich benachbart zu den jeweiligen kurzen Schnitten 811, 812 befindet, bildet einen Bandabschnitt. Dieser Bandabschnitt bildet eine Seilführungslasche 880 aus, wenn er zur Rohrmitte des Rohrelements gebogen wird, wie dies in Fig. 19 gezeigt ist. Ein Zugseil kann somit in dem gebildeten Zwischenraum zwischen der nach außen weisenden Fläche des Bandabschnittes der Seilführungslasche 880 und der in Längsrichtung benachbarten Innenumfangsfläche des Rohrelements geführt werden.

Die geraden Schnitte 801, 802 können dabei so am Biegeabschnitt A' vorgesehen werden, dass die Steifigkeit ähnlich wie im ersten Ausführungsbeispiel ist.

Durch das Vorsehen der geraden Schnitte 801, 802, 811, 812 kann die Maschinenlaufzeit, die für die Herstellung der Gelenke und Seilführungslaschen benötigt wird, massiv gesenkt werden. Dadurch werden die Produktionskosten gesenkt.

### Weitere Alternativen

Im Ausführungsbeispiel hat der flexible Abschnitt 20 unter Betrachtung in proximaler Richtung eine erste Zone B, eine zweite Zone C und eine dritte Zone D mit unterschiedlicher Flexibilität. Die Anzahl an Zonen oder Bereichen mit unterschiedlicher Flexibilität ist nicht beschränkt. Der flexible Abschnitt 20 kann auch mehr oder weniger Zonen mit unterschiedlicher Flexibilität haben. Die Erfindung ist auch auf einen Einführschlauch anwendbar, bei dem der flexible Abschnitt 20 eine durchgehend gleichbleibende Flexibilität besitzt.

Im Ausführungsbeispiel ist das Rohrelement des Einführschlauches 2 aus Edelstahl gebildet. Die Erfindung ist nicht darauf beschränkt. Das Material des Einführschlauches 2 kann ein beliebiges ausreichend steifes Material sein, wie z.B. ein steifer Kunststoff. In einer weiteren Alternative kann Nitinol (eine Nickel-Titan-Legierung) als Rohrmaterial angewendet werden. Dieses Material hat u.a. die Eigenschaft einer sogenannten Superelastizität, d.h. es kann in weiten Bereichen elastisch verformt werden, ohne sich bleibend zu verbiegen.

In einer Alternative werden durch eine Laserschneidmaschine im Rohrelement Schnitte vorgesehen. Diese Schnitte können sehr präzise vorgesehen werden. Daher wird eine Herstellung per Laser bevorzugt. Im Prinzip ist es jedoch denkbar, dass diese Schnitte auch durch andere Herstellverfahren wie z.B. Sägen, Drahtsägen etc. gefertigt werden.

Im Ausführungsbeispiel kann der Abwinkelungsabschnitt A in zwei Abwinkelungsrichtungen abgewinkelt werden, nämlich in den Figuren 6 und 7 nach oben und unten. In einer Alternative können die einzelnen Gelenkglieder 6 so ausgebildet sein, dass ihre Köpfe 622 von Gelenkglied 6 zu Gelenkglied 6 um 90 Grad gedreht um die Achse des Abwinkelungsabschnittes A (Achse der Gelenkglieder 6) versetzt sind. In dieser Alternative kann der Abwinkelungsabschnitt A in vier Abwinkelungsrichtungen abgewinkelt werden, nämlich in den Figuren 6 und 7 nach oben und unten und zum Betrachter hin und vom Betrachter weg.

In der Alternative, in der der Abwinkelungsabschnitt A in vier Abwinkelungsrichtungen abgewinkelt werden kann, können zwei Steuerdrähte 9 verwendet werden, die um 90 Grad zueinander versetzt im Einführschlauch 2 verlaufen. Das Gelenkglied 92 ist dann mit vier distalen Schlitzen versehen, die ebenfalls um 90 Grad zueinander versetzt sind.

Im Ausführungsbeispiel ist ein jeweiliges Gelenkglied 6 in der beschriebenen Form ausgebildet. Die Erfindung ist nicht auf die Form des Gelenkgliedes 6 beschränkt. Es ist ausreichend, wenn im Abwinkelungsabschnitt A Gelenkglieder geschnitten werden, die miteinander gekuppelt sind und eine Auslenkbewegung des Abwinkelungsabschnittes A ermöglichen.

Die Erfindung ist bei einem Duodenoskop, einem Gastroskop, einem Colonoskop oder einem ähnlichen Endoskop vorteilhaft anwendbar. Das Prinzip der Erfindung kann auch bei einer beliebigen anderen Art an Endoskop angewendet werden.

Das Prinzip der Erfindung ist auch bei anderen medizinischen Vorrichtungen anwendbar, die einen Einführschlauch verwenden.

### Bezugszeichenliste

- 1: Endoskop
- 2: Einführschlauch
- 3: Kontrollkörper
- 6: Gelenkglied
- 8: Führungsfeder
- 9: Steuerdraht
- 20: flexibler Abschnitt
- 61: Gelenkglied
- 62: Gelenkglied
- 63: Gelenkglied
- 69: am weitesten an der distalen Seite befindliches Gelenkglied
- 70: Schnitt
- 71: Scharnier
- 72: Lasche
- 73: unteres Ohr
- 74: oberes Ohr 74
- 75: Laschenmittelstück
- 77: Loch
- 91: erster Abschnitt des Steuerdrahtes
- 92: zweiter Abschnitt des Steuerdrahtes
- 93: dritter Abschnitt des Steuerdrahtes
- 201: Schnitt von oben
- 202: Schnitt von unten
- 203: nicht geschnittener Zwischenraum
- 204: Schnitt von der Seite
- 601: Kopflinie
- 602: Halslinie
- 603: Schulterlinie
- 604: Armlinie
- 605: Armendlinie
- 606: gebogene Fußlinie
- 607: Bodenlinie
- 608: gerade Fußlinie
- 609: Bauchlinie
- 621: Hauptkörper
- 622: Kopf
- 623: Arm
- 624: Fuß
- 630: Seilführungslasche
- 631: zentrisches Loch
- 691: Schlitz
- 692: Schlitz
- 801: Schnitt von oben
- 802: Schnitt von unten
- 803: nicht geschnittener Zwischenraum
- 805: Ringabschnitt mit kurzen Schnitten
- 811: kurzer Schnitt von oben
- 812: kurzer Schnitt von unten
- 880: Seilführungslasche
- A: Abwinkelungsabschnitt
- A': Abwinkelungsabschnitt
- B: erste Zone (distaler Bereich)
- C: zweite Zone (mittlerer Bereich)
- D: dritte Zone (proximaler Bereich)
- F: erstes Steuerrad (erstes Steuerelement)
- G: zweites Steuerrad (zweites Steuerelement)
- J: Kontrollkörpergehäuse
- K: Übergangsbereich
- S: seitlicher Einschnitt am flexiblen Abschnitt

## Patentansprüche

1. Verfahren zur Herstellung eines Einführschlauches (2) eines Endoskops,
wobei der Einführschlauch (2) einen proximalen passiven flexiblen Abschnitt (20) und einen distalen Abwinkelungsabschnitt (A) aufweist,
der passive flexible Abschnitt (20) und der Abwinkelungsabschnitt (A) einstückig sind und
einzelne Schnitte (201, 202, 801, 802) im als rohrartiges Element vorgesehenen flexiblen Abschnitt (20) so erzeugt werden, dass benachbarte einzelne Schnitte (201, 202, 801, 802) zueinander gleiche Abstände haben, **dadurch gekennzeichnet, dass**
an eine vordefinierte Anzahl der an in Längsrichtung des rohrartigen Elements hintereinander liegenden einzelnen Schnitte (801, 802) ein einzelner erster kurzer Schnitt (811) angrenzt, der kürzer als die anderen einzelnen Schnitte (801, 802) ist,
gegenüberliegend zum ersten kurzen Schnitt (811) ein zweiter kurzer Schnitt (812) ausgebildet wird, wobei der erste kurze Schnitt (811) und der zweite kurze Schnitt (812) ein Paar bilden und auf einer Ebene liegen,
ein Bandabschnitt des Materials des rohrartigen Elements zwischen dem ersten kurzen Schnitt (811) und dem benachbarten anderen einzelnen Schnitt (801, 802) der vordefinierten Anzahl an hintereinander liegenden einzelnen Schnitten (801, 802) zur Rohrmitte des rohrartigen Elements gebogen wird zur Ausbildung einer Seilführungslasche (880), und
ein Bandabschnitt des Materials des rohrartigen Elements zwischen dem zweiten kurzen Schnitt (812) und dem benachbarten anderen einzelnen Schnitt (801, 802) der vordefinierten Anzahl an hintereinander liegenden einzelnen Schnitten (801, 802) zur Rohrmitte des rohrartigen Elements gebogen wird zur Ausbildung einer Seilführungslasche (880).

2. Verfahren gemäß Anspruch 1, wobei
die einzelnen Schnitte (201, 202) im als rohrartiges Element vorgesehenen flexiblen Abschnitt (20) durch Laserschneiden erzeugt werden.

3. Verfahren gemäß Anspruch 1 oder 2, wobei
benachbarte Schnitte der einzelnen Schnitte (201, 202) entlang der Achse des flexiblen Abschnittes (20) in Längsrichtung des Einführschlauches (2) um 180 Grad versetzt sind.

4. Verfahren gemäß Anspruch 1 oder 2, wobei
benachbarte Schnitte der einzelnen Schnitte (201, 202) entlang der Achse des flexiblen Abschnittes (20) in Längsrichtung des Einführschlauches (2) um 90 Grad versetzt sind.

5. Verfahren gemäß einem der Ansprüche 1 bis 4, wobei
die einzelnen Schnitte (201, 202) im als rohrartiges Element vorgesehenen flexiblen Abschnitt (20) unter 180 Grad in bezug auf die Achse des flexiblen Abschnittes (20) erzeugt werden.

6. Verfahren gemäß einem der Ansprüche 1 bis 5, wobei
das gesamte Rohrelement des Einführschlauches (2) durch Laserschneiden hergestellt wird.

7. Verfahren gemäß einem der Ansprüche 1 bis 6, wobei
der proximale passive flexible Abschnitt (20) durch jeweilige seitliche Einschnitte (S), die senkrecht zur Längserstreckung des Rohrelementes erfolgen, erzeugt wird.

8. Endoskop mit einem Einführschlauch,
wobei der Einführschlauch (2) einen proximalen passiven flexiblen Abschnitt (20) und einen distalen Abwinkelungsabschnitt (A) aufweist,
der passive flexible Abschnitt (20) und der Abwinkelungsabschnitt (A) einstückig sind und
der als rohrartiges Element vorgesehene flexible Abschnitt (20) einzelne Schnitte (201, 202, 801, 802) aufweist, wobei benachbarte einzelne Schnitte (201, 202, 801, 802) zueinander gleiche Abstände haben,
**dadurch gekennzeichnet, dass**
an eine vordefinierte Anzahl der an in Längsrichtung des rohrartigen Elements hintereinander liegenden einzelnen Schnitte (801, 802) ein einzelner erster kurzer Schnitt (811) angrenzt, der kürzer als die anderen einzelnen Schnitte (801, 802) ist,
gegenüberliegend zum ersten kurzen Schnitt (811) ein zweiter kurzer Schnitt (812) ausgebildet ist, wobei der erste kurze Schnitt (811) und der zweite kurze Schnitt (812) ein Paar bilden und auf einer Ebene liegen,
ein Bandabschnitt des Materials des rohrartigen Elements zwischen dem ersten kurzen Schnitt (811) und dem benachbarten anderen einzelnen Schnitt (801, 802) der vordefinierten Anzahl an hintereinander liegenden einzelnen Schnitten (801, 802) zur Rohrmitte des rohrartigen Elements gebogen ist zur Ausbildung einer Seilführungslasche (880), und
ein Bandabschnitt des Materials des rohrartigen Elements zwischen dem zweiten kurzen Schnitt (812) und dem benachbarten anderen einzelnen Schnitt (801, 802) der vordefinierten Anzahl an hintereinander liegenden einzelnen Schnitten (801, 802) zur Rohrmitte des rohrartigen Elements gebogen ist zur Ausbildung einer Seilführungslasche (880).

9. Endoskop gemäß Anspruch 8, wobei
die einzelnen Schnitte (201, 202) im als rohrartiges Element vorgesehenen flexiblen Abschnitt (20) in einer Genauigkeit erzeugt sind, die durch Laserschneiden erreichbar ist.

10. Endoskop gemäß Anspruch 8 oder 9, wobei
benachbarte Schnitte der einzelnen Schnitte (201, 202) entlang der Achse des flexiblen Abschnittes (20) in Längsrichtung des Einführschlauches (2) um 180 Grad versetzt sind.

11. Endoskop gemäß Anspruch 8 oder 9, wobei
benachbarte Schnitte der einzelnen Schnitte (201, 202) entlang der Achse des flexiblen Abschnittes (20) in Längsrichtung des Einführschlauches (2) um 90 Grad versetzt sind.

12. Endoskop gemäß einem der Ansprüche 8 bis 11, wobei
die einzelnen Schnitte (201, 202) im als rohrartiges Element vorgesehenen flexiblen Abschnitt (20) sich unter 180 Grad in bezug auf die Achse des flexiblen Abschnittes (20) erstrecken.

13. Endoskop gemäß einem der Ansprüche 8 bis 12, wobei die einzelnen Schnitte (201, 202) gerade Schnitte sind.

14. Endoskop gemäß einem der Ansprüche 8 bis 13, wobei
das gesamte Rohrelement des Einführschlauches (2) in einer Genauigkeit ausgebildet ist, die durch Laserschneiden erreichbar ist.

15. Endoskop gemäß einem der Ansprüche 8 bis 14, wobei
der proximale passive flexible Abschnitt (20) jeweilige seitliche Einschnitte (S) aufweist, die sich senkrecht zur Längserstreckung des Rohrelementes erstrecken.

## Claims

1. A method for manufacturing an endoscope insertion tube (2),
wherein the insertion tube (2) has a proximal passive flexible section (20) and a distal angled section (A),
the passive flexible section (20) and the angled section (A) are integrally formed and
individual cuts (201, 202, 801, 802) are made in the flexible section (20), provided as a tube-like element, in such a way that adjacent individual cuts (201, 202, 801, 802) are equidistant from one another, **characterized in that**
at a predefined number of the individual cuts (801, 802) that lie behind each other in longitudinal direction of the tube-like element, a single first short cut (811) adjoins that is shorter than the other individual cuts (801, 802),
opposite to the first short cut (811), a second short cut (812) is formed, wherein the first short cut (811) and the second short cut (812) form a pair and lie on the same plane,
a band portion of the material of the tube-like element between the first short cut (811) and the adjacent other individual cut (801, 802) of the predefined number of the individual cuts (801, 802) that lie behind each other, is bend to the tube center of the tube-like element for forming a cable guide lug (880), and
a band portion of the material of the tube-like element between the second short cut (812) and the adjacent other individual cut (801, 802) of the predefined number of the individual cuts (801, 802) that lie behind each other, is bend to the tube center of the tube-like element for forming a cable guide lug (880).

2. The method according to claim 1, wherein
the individual cuts (201, 202) are made in the flexible section (20), provided as a tube-like element, by laser cutting.

3. The method according to claim 1 or 2, wherein
cuts adjacent to the individual cuts (201, 202) are offset by 180 degrees along the axis of the flexible section (20) in the longitudinal direction of the insertion tube (2).

4. The method according to claim 1 or 2, wherein
cuts adjacent to the individual cuts (201, 202) are offset by 90 degrees along the axis of the flexible section (20) in the longitudinal direction of the insertion tube (2).

5. The method according to one of claims 1 to 4, wherein
the individual cuts (201, 202) are made in the flexible section (20), provided as a tube-like element, at an angle of 180 degrees in relation to the axis of the flexible section (20).

6. The method according to one of claims 1 to 5, wherein
the overall tube element of the insertion tube (2) is manufactured by laser cutting.

7. The method according to one of claims 1 to 6, wherein
the proximal passive flexible section (20) is produced by respective lateral indentations (S) that are provided perpendicular to the longitudinal extension of the tube element.

8. An endoscope having an insertion tube,
wherein the insertion tube (2) has a proximal passive flexible section (20) and a distal angled section (A),
wherein the passive flexible section (20) and the angled section (A) are integrally formed and
the flexible section (20), provided as a tube-like element, has individual cuts (201, 202, 801, 802), wherein adjacent individual cuts (201, 202, 801, 802) are equidistant from one another,
**characterized in that**
at a predefined number of the individual cuts (801, 802) that lie behind each other in longitudinal direction of the tube-like element, a single first short cut (811) adjoins that is shorter than the other individual cuts (801, 802),
opposite to the first short cut (811), a second short cut (812) is formed, wherein the first short cut (811) and the second short cut (812) form a pair and lie on the same plane,
a band portion of the material of the tube-like element between the first short cut (811) and the adjacent other individual cut (801, 802) of the predefined number of the individual cuts (801, 802) that lie behind each other, is bend to the tube center of the tube-like element for forming a cable guide lug (880), and
a band portion of the material of the tube-like element between the second short cut (812) and the adjacent other individual cut (801, 802) of the predefined number of the individual cuts (801, 802) that lie behind each other, is bend to the tube center of the tube-like element for forming a cable guide lug (880).

9. The endoscope according to claim 8, wherein
the individual cuts (201, 202) are made in the flexible section (20), provided as a tube-like element, with an accuracy that is achievable by laser cutting.

10. The endoscope according to claim 8 or 9, wherein
cuts adjacent to the individual cuts (201, 202) are offset by 180 degrees along the axis of the flexible section (20) in the longitudinal direction of the insertion tube (2).

11. The endoscope according to claim 8 or 9, wherein
cuts adjacent to the individual cuts (201, 202) are offset by 90 degrees along the axis of the flexible section (20) in the longitudinal direction of the insertion tube (2).

12. The endoscope according to one of claims 8 to 11, wherein
the individual cuts (201, 202) extend in the flexible section (20), provided as a tube-like element, at an angle of 180 degrees in relation to the axis of the flexible section (20).

13. The endoscope according to one of claims 8 to 12, wherein
the individual cuts (201, 202) are straight cuts.

14. The endoscope according to one of claims 8 to 13, wherein
the overall tube element of the insertion tube (2) is formed with an accuracy that is achievable by laser cutting.

15. The endoscope according to one of claims 8 to 14, wherein
the proximal passive flexible section (20) has respective lateral indentations (S) that extend perpendicularly with respect to the longitudinal extension of the tube element.

## Revendications

1. Procédé de fabrication d'un tuyau d'insertion (2) d'un endoscope,
le tuyau d'insertion (2) possédant une portion flexible passive (20) proximale et une portion à coudage (A) distale,
la portion flexible passive (20) et la portion à coudage (A) étant d'un seul tenant et
des coupes individuelles (201, 202, 801, 802) étant générées dans la portion flexible (20) présente sous la forme d'un élément de type tubulaire de telle sorte que des coupes individuelles (201, 202, 801, 802) voisines possèdent respectivement les mêmes écarts entre elles,
**caractérisé en ce que**
une première coupe courte (811) individuelle, laquelle est plus courte que les autres coupes individuelles (801, 802), est adjacente à un nombre prédéfini des coupes individuelles (801, 802) se trouvant les unes derrière les autres dans la direction longitudinale de l'élément de type tubulaire,
une deuxième coupe courte (812) est formée à l'opposé de la première coupe courte (811), la première coupe courte (811) et la deuxième coupe courte (812) formant une paire et se trouvant sur un plan,
une portion en bande du matériau de l'élément de type tubulaire entre la première coupe courte (811) et l'autre coupe individuelle (801, 802) voisine du nombre prédéfini de coupes individuelles (801, 802) se trouvant les unes derrière les autres est courbée vers le centre de tube de l'élément de type tubulaire en vue de former une languette de guidage de câble (880), et
une portion en bande du matériau de l'élément de type tubulaire entre la deuxième coupe courte (812) et l'autre coupe individuelle (801, 802) voisine du nombre prédéfini de coupes individuelles (801, 802) se trouvant les unes derrière les autres est courbée vers le centre de tube de l'élément de type tubulaire en vue de former une languette de guidage de câble (880).

2. Procédé selon la revendication 1,
les coupes individuelles (201, 202) dans la portion flexible (20) présente sous la forme d'un élément de type tubulaire étant produites par découpe au laser.

3. Procédé selon la revendication 1 ou 2,
les coupes voisines des coupes individuelles (201, 202) le long de l'axe de la portion flexible (20) dans la direction longitudinale du tuyau d'insertion (2) étant décalées de 180 degrés.

4. Procédé selon la revendication 1 ou 2,
les coupes voisines des coupes individuelles (201, 202) le long de l'axe de la portion flexible (20) dans la direction longitudinale du tuyau d'insertion (2) étant décalées de 90 degrés.

5. Procédé selon l'une des revendications 1 à 4,
les coupes individuelles (201, 202) dans la portion flexible (20) présente sous la forme d'un élément de type tubulaire étant produites à moins de 180 degrés par rapport à l'axe de la portion flexible (20).

6. Procédé selon l'une des revendications 1 à 5,
la totalité de l'élément tubulaire du tuyau d'insertion (2) étant produit par découpe au laser.

7. Procédé selon l'une des revendications 1 à 6,
la portion flexible passive (20) proximale étant produite par des entailles (S) latérales respectives qui sont effectuées perpendiculairement à l'extension longitudinale de l'élément tubulaire.

8. Endoscope comprenant un tuyau d'insertion,
le tuyau d'insertion (2) possédant une portion flexible passive (20) proximale et une portion à coudage (A) distale,
la portion flexible passive (20) et la portion à coudage (A) étant d'un seul tenant et
la portion flexible (20) présente sous la forme d'un élément de type tubulaire possédant des coupes individuelles (201, 202, 801, 802), les coupes individuelles (201, 202, 801, 802) voisines possédant les mêmes écarts entre elles,
**caractérisé en ce que**
une première coupe courte (811) individuelle, laquelle est plus courte que les autres coupes individuelles (801, 802), est adjacente à un nombre prédéfini des coupes individuelles (801, 802) se trouvant les unes derrière les autres dans la direction longitudinale de l'élément de type tubulaire,
une deuxième coupe courte (812) est formée à l'opposé de la première coupe courte (811), la première coupe courte (811) et la deuxième coupe courte (812) formant une paire et se trouvant sur un plan,
une portion en bande du matériau de l'élément de type tubulaire entre la première coupe courte (811) et l'autre coupe individuelle (801, 802) voisine du nombre prédéfini de coupes individuelles (801, 802) se trouvant les unes derrière les autres est courbée vers le centre de tube de l'élément de type tubulaire en vue de former une languette de guidage de câble (880), et
une portion en bande du matériau de l'élément de type tubulaire entre la deuxième coupe courte (812) et l'autre coupe individuelle (801, 802) voisine du nombre prédéfini de coupes individuelles (801, 802) se trouvant les unes derrière les autres est courbée vers le centre de tube de l'élément de type tubulaire en vue de former une languette de guidage de câble (880).

9. Endoscope selon la revendication 8,
les coupes individuelles (201, 202) dans la portion flexible (20) présente sous la forme d'un élément de type tubulaire étant produites avec une précision qui peut être atteinte par découpe au laser.

10. Endoscope selon la revendication 8 ou 9,
les coupes voisines des coupes individuelles (201, 202) le long de l'axe de la portion flexible (20) dans la direction longitudinale du tuyau d'insertion (2) étant décalées de 180 degrés.

11. Endoscope selon la revendication 8 ou 9,
les coupes voisines des coupes individuelles (201, 202) le long de l'axe de la portion flexible (20) dans la direction longitudinale du tuyau d'insertion (2) étant décalées de 90 degrés.

12. Endoscope selon l'une des revendications 8 à 11,
les coupes individuelles (201, 202) dans la portion flexible (20) présente sous la forme d'un élément de type tubulaire s'étendant sur moins de 180 degrés par rapport à l'axe de la portion flexible (20).

13. Endoscope selon l'une des revendications 8 à 12,
les coupes individuelles (201, 202) sont des coupes droites.

14. Endoscope selon l'une des revendications 8 à 13,
la totalité de l'élément tubulaire du tuyau d'insertion (2) étant formé avec une précision qui peut être atteinte par découpe au laser.

15. Endoscope selon l'une des revendications 8 à 14,
la portion flexible passive (20) proximale possédant des entailles (S) latérales respectives qui s'étendent perpendiculairement à l'extension longitudinale de l'élément tubulaire.
